# EUROPÄISCHE PATENTSCHRIFT

(11) **EP 0 844 227 B1**
(45) Veröffentlichungstag und Bekanntmachung des Hinweises auf die Patenterteilung: **16.08.2001**
(21) Anmeldenummer: 97119770.2
(22) Anmeldetag: 12.11.1997
(51) Int. Cl.: C07C 29/60, C07C 33/025, B01J 27/16, B01J 27/18

(54) **Verfahren zur Herstellung von Alpha,Omega-C4- bis C20-Alkenolen**
Method for the preparation of alpha,omega C4 to C20 alkenols
Procédé de préparation d'alpha-oméga alcénols en C4 à C20

(30) Priorität: 25.11.1996 DE 19648637
(43) Veröffentlichungstag der Anmeldung: 27.05.1998
(73) Patentinhaber: Goldschmidt AG, 45127 Essen (DE)
(72) Erfinder: Eufinger, Jörg, 45476 Mülheim (DE); Knott, Wilfried, Dr., 45141 Essen (DE); Mehrwald, Andreas, Dr., 45307 Essen (DE); Provinzano, Angelo, 46242 Bottrop (DE)

(56) Entgegenhaltungen:
- EP-A- 0 015 318
- EP-A- 0 195 943

## Beschreibung

Die Erfindung betrifft ein Verfahren zur Herstellung von α,ω-C₄- bis C₂₀-Alkenolen durch katalytische Gasphasendehydratisierung von α,ω-Alkandiolen.

Zur Synthese von α,ω-C₄- bis C₂₀-Alkenolen mit Reinheitsgraden über 85 % durch katalytische Dehydratisierung der entsprechenden α,ω-C₄- bis C₂₀-Diole setzt man nach einem Verfahren der DE-C-29 04 518 neutrale, einfache oder Misch-Pyrophosphate des Lithiums, Natriums, Strontiums oder Bariums bzw. Gemische dieser Verbindungen als Katalysator ein. Der Nachteil dieses Verfahrens liegt zum einen in der Bildung merklicher Anteile von Alkanolen, zum anderen steigt die Bildung anderer Nebenprodukte bei Umsätzen von > 80 merklich an und bei Umsätzen von ≥ 90 % erheblich.

Die EP-B-0 195 943 greift diesen Stand der Technik auf und lehrt ein Verfahren zur Herstellung von reinen α,ω-C₄- bis C₂₀-Alkenolen mit Reinheitsgraden über 90 % durch katalytische Dehydratisierung der entsprechenden α,ω-C₄- bis C₂₀-Diole, welches dadurch gekennzeichnet ist, daß man α,ω-C₄- bis C₂₀-Diole mit Hilfe von mit Alkali- oder Erdalkali-Verbindungen dotierten einfachen oder Misch-Phosphaten der Elemente der 2. Hauptgruppe als Katalysatoren, wobei man durch Einsatz von Erdalkaliorthophosphat oder Erdalkalihydrogenphosphat bzw. durch Umsetzung einer Erdalkaliverbindung mit Phosphorsäure zum entsprechenden Phosphat und Zusatz einer Alkali- oder Erdalkaliverbindung eine Katalysatormasse herstellt, diese gegebenenfalls verformt, anschließend trocknet und sie dann bei Temperaturen von 350 bis 950 °C calciniert, bei Temperaturen von 300 bis 500 °C selektiv und partiell zu α,ω-C₄- bis C₂₀-Alkenolen mit Reinheitsgraden über 90 % bei Umsätzen von über 90 % dehydratisiert.

Die eingesetzten Katalysatoren genügen unter den Gesichtspunkten Selektivität und Standzeiten technischen Erfordernissen, bieten aber unter dem produktionstechnisch bedeutsamen Aspekt des Mengendurchsatzes, d. h. der effektiven Katalysatorbelastbarkeit bei reduzierter Verweilzeit keine befriedigende Lösung. Als Maß für die effektive Katalysatorbelastung wird häufig der LHSV-Wert angegeben, der das Verhältnis von Eduktstrom zu Katalysatorvolumen wiedergibt. Gemäß EP-B-0 195 943 werden für das beschriebene Verfahren für die Katalysatorbelastung LHSV-Werte zwischen 0,25 und 0,45 ml/(ml · h) angegeben.

Ziel der vorliegenden Erfindung ist es, eine hohe Selektivität verknüpft mit großer Raum/Zeit-Ausbeute bei der Gasphasendehydratisierung von α,ω-Alkandiolen, speziell 1,6-Hexandiol, durch Auswahl eines geeigneten Katalysatorsystems zu gewährleisten.

Überraschenderweise wurde gefunden, daß ein Mischkatalysator, bestehend aus Bariumphosphat und Aluminiumphosphat, sowohl eine große katalytische Aktivität als auch eine excellente Selektivität aufweist.

Gegenstand der Erfindung ist somit ein Verfahren zur Herstellung von α,ω-C₄- bis C₂₀-Alkenolen, vorzugsweise α,ω-C₆- bis C₁₄-Alkenolen, durch katalytische Monodehydratisierung der entsprechenden Alkandiole bei Temperaturen von 300 bis 500 °C, mit dem Kennzeichen, daß man als Katalysator einen Aluminiumphosphat/Bariumphosphat-Mischkatalysator einsetzt.

Dabei wird das Aluminiumphosphat/Bariumphosphat-System im Molverhältnis 1 : 10 bis 10 : 1, insbesondere 1 : 5 bis 5 : 1 eingesetzt.

Der erfindungsgemäß eingesetzte Aluminiumphosphat/Bariumphosphat-Mischkatalysator kann in an sich bekannter Weise, z. B. durch intensives Vermischen und Kompaktieren von Aluminium- und Bariumphosphat, hergestellt werden.

Der Mischkatalysator wird in besonders einfacher Weise erhalten, indem man Aluminiumphosphat mit Bariumcarbonat unter intensiver Vermischung mit Phosphorsäure versetzt, die erhaltene Salzmasse mit Wasser zur besseren Verformbarkeit anteigt, verformt, danach trocknet und bei Temperaturen von 400 bis 600°C calciniert.

Abweichend von der technischen Lehre der EP-B-0 195 943 bedeutet die Herstellmethode des erfindungsgemäßen Katalysators eine wesentliche Vereinfachung, da man auf die dort notwendige, ganz bestimmte Weise der Zudosierung von Katalysatorbestandteilen mit vorgegebenen Geschwindigkeiten verzichten kann.

Die Zusammensetzung des erfindungsgemäßen Kontaktes ist gegeben durch das molare Verhältnis von Aluminiumphosphat zu Bariumphosphat im Bereich von 1 : 10 bis 10 : 1, insbesondere 1 : 5 bis 5 : 1.

Vorteilhaft bei der Durchführung des erfindungsgemäßen Verfahrens ist die Zuspeisung von Wasser, vorzugsweise in Mengen von 10 bis 60 Gew.-%, als Quasi-Inertstoff in den Eduktstrom. Offenbar erhält die Anwesenheit von Wasserdampf in der Katalysatorzone die sauer-katalytische Aktivität des Kontaktes und wirkt zudem als thermischer Puffer, der die Isothermie der endothermen Eliminierung begünstigt. Insgesamt wird die Lebensdauer der verwendeten Kontaktmasse erhöht, da unerwünschte Verkokungs- und Crackprozesse in den Hintergrund gedrängt werden.

Im Falle der mit Wasser mischbaren Diole (z. B. Hexandiol-1,6) kann man unter Einsparung der Schmelzwärme die Lösung des Diols in Wasser einfach aus dem Vorlagegefäß in den Verdampfer und Überhitzer fördern.

Die nicht mit Wasser mischbaren Diole werden vorzugsweise in der Vorlage aufgeschmolzen und dann dem System, bestehend aus Verdampfer und Überhitzer, zugeführt. Vor Eintritt in den Reaktor können die nunmehr gasförmigen Diole mit Wasserdampf beaufschlagt werden.

Prinzipiell ist aber auch eine Ausführung des Verfahrens denkbar, bei der man, der Wasserdampf-Destillation analog, die Diole mit einem Wasserdampfstrom aus einer Vorlage in den Verdampfer und Überhitzer hineintreibt.

Im direkten Vergleich mit Katalysatoren gemäß EP-B-0 195 943 zur Dehydratisierung von α,ω-Diolen ist der erfindungsgemäß eingesetzte Ba/Al-Mischkatalysator durch seine große Aktivität geeignet, sehr kurze Kontaktzeiten in der Reaktionszone zu realisieren. Als vergleichende Kenngrößen, die nicht apparatespezifisch sind, eignen sich zur Charakterisierung der jeweiligen Katalysatorgüte der Umsatz, die Selektivität und der oben bereits genannte LHSV-Wert.

Die vergleichende Betrachtung der Gasphasendehydratisierung von 1,6-Hexandiol an Katalysatoren gemäß EP-B-0 195 943 und dem erfindungsgemäßen Katalysatorsystem belegt (s. Tabelle), daß bei Verwendung des neuen Katalysators unter der Aufrechterhaltung vergleichbarer Umsätze und Selektivitäten der LHSV-Wert, als ein direktes Maß für die effektive Katalysatorbelastbarkeit, wesentlich erhöht werden kann, was sich in einer erheblich gesteigerten Raum/Zeit-Ausbeute (= Mol Produkt/l Katalysatorvolumen und Stunde) niederschlägt.

Diese Aussage gewinnt für die Umsetzung dieser Reaktion in den technischen Maßstab an enormer Bedeutung, da sie dem Anlagenbetreiber gestattet, aus wesentlich kleineren Reaktorvolumina vergleichbare Produktströme auszutragen.

### Herstellung des Aluminiumphosphat-Bariumphosphat-Mischkontaktes

### Beispiel A

54 g (2 Mol) Aluminiumgrieß werden unter Rühren portionsweise mit 230,6 g (2 Mol) 85 %iger Phosphorsäure versetzt. Nach Abklingen der Reaktion wird der feuchte Salzkuchen für 12 Stunden bei 120 °C im Trockenschrank entwässert.

24,4 g AlPO₄ (0,2 Mol) werden zusammen mit 54,4 g (0,28 Mol) Bariumcarbonat intensiv in einem Mörser verrieben und dann innerhalb von 30 Minuten mit 61,0 g (0,53 Mol) konzentrierter Phosphorsäure versetzt. Zur Vervollständigung der Reaktion werden 50 ml Wasser ergänzt. Danach wird die gewonnene Salzmasse 30 Stunden bei 120 °C getrocknet.

Vor dem Einsatz wird der Katalysator für mindestens 4 Stunden bei 400 °C calciniert.

| Chemische Zusammensetzung: | |
|---|---|
| Al-Gehalt | 5,3 Gew.-% |
| Ba-Gehalt | 37,8 Gew.-% |
| P-Gehalt | 23,3 Gew.-% |

### Beispiel B

54 g (2 Mol) Aluminiumgrieß werden unter Rühren portionsweise mit 186 g (2 Mol) konzentrierter Phosphorsäure versetzt, wobei sich in stark exothermer Reaktion unter Wasserstoffentwicklung ein zäh-gelatinöses Aluminiumphosphat AlPO₄ bildet. Dieses Material wird für 12 Stunden bei 120 °C im Trockenschrank entwässert.

70,1 g AlPO₄ (0,58 Mol) werden zusammen mit 98,5 g (0,5 Mol) BaCO₃ in einem Mörser intensiv verrieben und dann innerhalb von 30 Minuten mit 32,3 g (0,33 Mol) konzentrierter Phosphorsäure versetzt. Nach beendeter Reaktion wird die Salzmasse mit Wasser angeteigt und zu zylindrischen Stäbchen verformt. Die Katalysatormasse wird 30 Stunden bei 120 °C getrocknet.

Vor dem Einsatz wird der Katalysator für mindestens 4 Stunden bei 400 °C calciniert.

| Chemische Zusammensetzung: | | |
|---|---|---|
| Al₂O₃-Gehalt | 19,90 Mol-% | 14,79 Gew.-% |
| BaO-Gehalt | 27,44 Mol-% | 30,70 Gew.-% |
| P₂O₅-Gehalt | 52,65 Mol-% | 54,50 Gew.-% |

| Physikalische Eigenschaften: | |
|---|---|
| Form | Stränge |
| Spez. Oberfläche (BET) | 2,3 m²/g |
| Schüttgewicht | 0,966 g/cm³ |

### Herstellungsbeispiele

### Beispiel 1

### Gasphasendehydratisierung von 1,6-Hexandiol Apparatebeschreibung

In der Apparatur zur Gasphasendehydratisierung von 1,6-Hexandiol wird ein auf 60 °C temperierter, gläserner Doppelmanteltropftrichter als Vorlage flüssigen Diols genutzt. Aus dieser Vorlage wird eine kleine Membranpumpe mit variabler Hublänge und -frequenz gespeist, die das flüssige Diol in ein spiralartig, zwischen zwei Flanschen gewundenes Rohr von 6 m Länge fördert, in dem sowohl Verdampfung als auch Überhitzung des Eduktes stattfinden. Der Außendurchmesser des Verdampfer- und Überhitzerrohres beträgt 4 mm bei einer Wandstärke von 1 mm (Dᵢ = 2 mm).

Der Bodenflansch der Apparatur ruht auf einer Laborheizplatte von 2 kW Leistung und leitet die thermische Energie direkt in das Verdampfer- und Überhitzerrohr. Dieses Rohr geht über in ein auf dem Flansch zentriertes, vertikal ausgerichtetes Stahlrohr (H = 80 mm, Dₐ = 25 mm, Dᵢ = 15 mm), das als Strömungsrohrreaktor dient und jeweils mit dem zu untersuchenden Katalysator gefüllt wird. Paßring-fixierte Metallsiebe am Reaktoreinund auslaß begrenzen die maximale Schütthöhe des Katalysatorbetts auf 60 mm.

Ein nachgeschalteter Kühler übernimmt die Kondensation und Abkühlung der aus der Reaktionszone entströmenden Reaktionsmischung und leitet über in eine Wechselvorlage zum Auffangen von Einzelfraktionen. Entweichende gasförmige Leichtsieder werden zusätzlich in einer mit Aceton/Trockeneis (-76 °C) gekühlten Kältefalle aufgefangen.

Die Temperaturmessung geschieht mittels am Reaktorein- und auslaß angebrachten Ni/Cr-Ni-Thermoelementen. Ein hinter der Förderpumpe installiertes Manometer zeigt den statischen Druck in der Eduktleitung an. Optionell gestattet eine zusätzliche Rohrleitung auf der Druckseite der Pumpe die Einspeisung von inerten gasförmigen oder flüssigen Stoffen.

Die Reaktionszone wird mittels einer um den Reaktor gewickelten Heizschnur (Pₑₗ = 150 W, Tₘₐₓ = 400 °C) elektrisch beheizt. Zur Gewährleistung einer isothermen Reaktionsführung durch Vermeidung von Wärmeverlusten ist die Apparatur mit Fibaflax (faserige Aluminiumoxid-Wolle) und Aluminiumfolie isoliert. Diese Maßnahmen gestatten eine isotherme Reaktionsführung mit einer maximalen Temperaturdifferenz von 5 K zwischen Reaktorein- und -austritt. Die Abweichung der mittleren Temperatur vom Sollwert liegt bei maximal 2 K.

Die aufgefangenen kondensierten Reaktionsprodukte werden mittels Gaschromatographie bestimmt und begleitend durch ¹H-NMR-Analytik charakterisiert.

### Versuchsbeschreibung

Der Rohrreaktor wird zunächst mit 10 g des Katalysators aus Beispiel 1 gefüllt und anschließend auf die gewünschte Temperatur (450 °C) aufgeheizt. Bei Erreichen einer konstanten Temperatur wird über die Dosierpumpe ein definierter Mengenstrom (1,95 ml/min) des in dem Doppelmanteltropftrichter verflüssigten Edukts (Gemisch aus 80 Gew.-% Hexandiol und 20 Gew.-% Wasser) über das Verdampfer- und Überhitzerrohr in den Reaktor geleitet. Die Temperatur wird so geregelt, daß eine isotherme Fahrweise gewährleistet ist. Das gasförmige Reaktionsprodukt wird nach Verlassen des Reaktors in dem Kühler sowie der nachgeschalteten Kühlfalle kondensiert, in der Wechselvorlage aufgefangen und anschließend mittels Gaschromatographie und ¹H-NMR-Analytik bestimmt. In dem beschriebenen Beispielversuch erhält man 34 Gew.-% 5-Hexen-1-ol sowie 28 Gew.-% nicht umgesetztes 1,6-Hexandiol, was einer Selektivität von 77,9 Mol-% und einem Umsatz von 64,9 % entspricht. Als Maß für die Bewertung der Katalysatorleistung ergibt sich daraus eine Raum/Zeit-Ausbeute von 40,28 Mol 5-Hexen-1-ol pro Liter Katalysatorvolumen und Stunde.

### Beispiele 2 und 3

Analog Beispiel 1 wurden 1,10-Decandiol bzw. 1,8-Octandiol umgesetzt. Die erhaltenen Ergebnisse sind in der nachfolgenden Tabelle aufgeführt.

### Beispiel 4

Hexandiol-1,6 wird in einem beheizten Vorlagegefäß aufgeschmolzen und dann in Analogie zu Beispiel 1 mit einem konstanten Volumenstrom von 1,5 ml/min durch die Dosierpumpe über Verdampfer und Überhitzer dem Reaktor zugeführt. Die Temperatur des Reaktors wird so geregelt, daß eine isotherme Fahrweise gewährleistet ist.

Gaschromatographie und NMR-Spektroskopie belegen die in der Tabelle dargestellten Parameter.

Der Versuch unterstreicht die positive Wirkung des dem Eduktstrom zugeführten Wassers (Beispiel 1), da bei vergleichbarer Katalysatorbelastung und nahezu identischem Umsatz eine geringere Selektivität erzielt wird.

**Tabelle**

| Kontakt | Temperatur °C | LHSV ml/(ml · h) | Umsatz Mol-% | Selektivität Mol-% | Ausbeute % | Raum/Zeit-Ausbeute Mol/(l · h) |
|---|---|---|---|---|---|---|
| B (EP-B-0 195 943) | 380 | 0,29 | 94,8 | 49,5 | 46,9 | 1,20 |
| C (EP-B-0 195 943) | 400 | 0,45 | 49,1 | 55 | 27 | 1,07 |
| II (EP-B-0 195 943) | 440 | 0,25 | 96,2 | 70 | 67,3 | 1,49 |
| erfindungsgem. Beispiel 1 1,6-Hexandiol | 451 | 9 | 64,9 | 77,9 | 50,6 | 40,28 |
| erfindungsgem. Beispiel 2 1,10-Decandiol | 460 | 9 | 60 | 48 | 29 | 15,74 |
| erfindungsgem. Beispiel 3 1,8-Octandiol | 457 | 7,8 | 69 | 62 | 43 | 24,09 |
| erfindungsgem. Beispiel 4 1,6-Hexandiol | 464 | 9 | 63 | 49,2 | 31 | 24,68 |

## Patentansprüche

1. Verfahren zur Herstellung von α,ω-C₄- bis C₂₀-Alkenolen durch katalytische Monodehydratisierung der entsprechenden Alkandiole bei Temperaturen von 300 bis 500 °C, dadurch gekennzeichnet, daß man als Katalysator einen Aluminiumphosphat/Bariumphosphat-Mischkatalysator einsetzt.

2. Verfahren gemäß Anspruch 1, dadurch gekennzeichnet, daß das Aluminiumphosphat/Bariumphosphat-System im Molverhältnis 1 : 10 bis 10 : 1, insbesondere 1 : 5 bis 5 : 1 eingesetzt wird.

3. Verfahren gemäß Anspruch 1 oder 2, dadurch gekennzeichnet, daß man den Katalysator bei Temperaturen von 400 bis 600 °C calciniert.

4. Verfahren gemäß den Ansprüchen 1 bis 3, dadurch gekennzeichnet, daß in den Eduktstrom Wasser zugespeist wird.

## Claims

1. Process for preparing α,ω-C₄-C₂₀-alkenols by catalytic monodehydration of the corresponding alkanediols at temperatures of from 300 to 500°C, characterized in that the catalyst used is an aluminium phosphate/barium phosphate mixed catalyst.

2. Process according to Claim 1, characterized in that the aluminium phosphate/barium phosphate system is used in a molar ratio of from 1:10 to 10:1, in particular from 1:5 to 5:1.

3. Process according to Claim 1 or 2, characterized in that the catalyst is calcined at temperatures of from 400 to 600°C.

4. Process according to Claims 1 to 3, characterized in that water is fed into the starting material flow.

## Revendications

1. Procédé pour la préparation de α,ω-(alcénols en C₄ à C₂₀) par monodéshydratation catalytique des alcanediols correspondants à des températures de 300°C à 500°C, caractérisé en ce qu'on utilise comme catalyseur un catalyseur mixte de phosphate d'aluminium et de phosphate de baryum.

2. Procédé selon la revendication 1, caractérisé en ce que le système phosphate d'aluminium et de phosphate de baryum est utilisé dans un rapport molaire de 1:10 à 10:1, en particulier de 1:5 à 5:1.

3. Procédé selon les revendications 1 ou 2, caractérisé en ce qu'on calcine le catalyseur à des températures de 400°C à 600°C.

4. Procédé selon les revendications 1 à 3, caractérisé en ce qu'on alimente le flux de produits de départ en eau.
